# NOUVEAU FASCICULE DE BREVET EUROPEEN

(11) **EP 2 349 203 B2**
(45) Date de publication et mention de la décision concernant l'opposition: **26.02.2020**
(45) Mention de la délivrance du brevet: 30.10.2013
(21) Numéro de dépôt: 09768146.4
(22) Date de dépôt: 04.11.2009
(51) Int. Cl.: A61K 9/00, A61K 31/167, A61K 9/08, A61K 47/12, A61K 47/10, A61K 47/18, A61K 8/03, A61Q 19/08, A61P 17/00

(54) **Composition injectable a base d'acide hyaluronique ou l'un de ses sels, de polyols et de lidocaïne, sterilisée à la chaleur**
Wärmesterilisierte Injizierbare Zusammensetzung von Hyaluronsäure oder eines ihrer Salze, Polyole und Lidocain
Heat sterilised injectable composition of hyaluronic acid or one of the salts thereof, polyols and lidocaine

(30) Priorité: 07.11.2008 FR 0857575
(43) Date de publication de la demande: 03.08.2011
(60) Demande divisionnaire: 13184851.7
(73) Titulaire: ANTEIS S.A., 1228 Plan-Les-Ouates, Genève (CH)
(72) Inventeur: GAVARD MOLLIARD, Samuel, F-74250 Bogeve (FR)
(74) Mandataire: Ricker, Mathias
(86) Numéro de dépôt international: PCT/FR2009/052134
(87) Numéro de publication internationale: WO 2010/052430

(56) Documents cités:
- WO-A-98/41171
- WO-A-2007/077399
- WO-A-2008/068297
- WO-A1-93/12801
- WO-A1-2005/067994
- WO-A1-2005/072751
- WO-A1-2006/067608
- WO-A1-2009/071697
- WO-A2-2004/072759
- WO-A2-2005/112888
- WO-A2-2006/122183
- WO-A2-2007/128923
- WO-A2-2008/088381
- WO-A2-2010/136694
- US-A1- 2007 036 745
- WAHL, G.: "European evaluation of a new hyaluronic acid filler incorporating lidocaine" JOURNAL OF COSMETIC DERMATOLOGY, vol. 7, 6 novembre 2008 (2008-11-06), pages 298-303, XP002516376
- "European Standard EN ISO 176651:2006", ,
- "The European Aesthetic Guide", 2008 pages 120-122,
- SCHECHTMANN, A.: "Combining Triamcinolone and Lidocaine for Soft Tissue Injections", Am Fam Physician., vol. 77, no. 10, 15 May 2008 (2008-05-15), page 1372,
- "Delivery note and data sheet re Stylage M of Vivacy", ,
- "Lidocaine"; "5503" In: "Merck Index, 13ed.", 2001, Whitehouse Station, NJ. page 982,

## Description

L'invention concerne une composition aqueuse injectable sous forme de gel, utilisée dans des buts esthétiques ou dans des buts thérapeutiques, à base d'acide hyaluronique ou l'un de ses sels, d'un ou plusieurs polyol(s) et de lidocaïne, ayant subi une stérilisation à la chaleur qui a pour effets une amélioration des propriétés rhéologiques de viscoélasticité et une amélioration de la rémanence in vivo, par rapport à un gel sans polyol et sans lidocaïne, caractérisée en ce que le gel à base d'acide hyaluronique ou l'un de ses sels est réticulé.

Les gels injectables à base d'acide hyaluronique sont utilisés depuis de nombreuses années dans des buts esthétiques pour le comblement ou le remplacement de tissus biologiques (comblement des rides, remodelage du visage, augmentation du volume des lèvres, ...) ainsi que dans le traitement de réhydratation de la peau par mésothérapie.

Les gels injectables à base d'acide hyaluronique sont également utilisés dans de nombreuses applications thérapeutiques. Par exemple,
- en rhumatologie, en tant qu'agent de remplacement, de supplément temporaire du liquide synovial,
- en urologie/gynécologie, en tant qu'agent permettant d'augmenter le volume du sphincter ou de l'urètre,
- en ophtalmologie, en tant qu'adjuvant à la chirurgie de la cataracte ou pour le traitement des glaucomes,
- en pharmaceutique, en tant que gel à libération de substances actives,
- en chirurgie, pour la reconstruction osseuse, l'augmentation du volume des cordes vocales ou la fabrication de tissus chirurgicaux.

De nombreux efforts ont été faits pour améliorer la stabilité physico-chimique des gels à base d'acide hyaluronique afin d'augmenter leur rémanence in vivo (c'est-à-dire le temps de résidence du gel au niveau du site d'injection) et ainsi, augmenter la durée d'efficacité des traitements.

D'après l'art antérieur, l'augmentation de la rémanence des gels à base d'acide hyaluronique et donc de leur résistance aux différents facteurs de dégradation in vivo est essentiellement mise en oeuvre par des techniques de réticulation et/ou greffage de l'acide hyaluronique. Par exemple,
- WO 2005/012364 décrit des gels à base de polysaccharides, dont l'acide hyaluronique, réticulés et greffés, ayant une meilleure rémanence que les produits non réticulés et/ou non greffés.
- WO 2004/092222 décrit des gels à base de polysaccharides, dont l'acide hyaluronique, renfermant au moins un polysaccharide de faible masse moléculaire et au moins un polysaccharide de forte masse moléculaire, gels ayant une rémanence supérieure à celle de produits ne possédant pas de mélange de masses moléculaires.
- WO 2005/085329 décrit un procédé de fabrication permettant l'obtention de gels à base d'acide hyaluronique réticulé polydensifié, gels possédant une longue rémanence in vivo.
- WO 2000/0046252 décrit des gels à base d'acide hyaluronique à forte « biostabilité » possédant un haut degré de réticulation grâce à un procédé de double réticulation de l'acide hyaluronique.

Les polyols appartiennent à une famille de molécules de formule chimique CₓHyOz qui possèdent au moins deux groupements alcool. De par leur forte capacité à ajuster l'osmolarité, l'homme de l'art sait qu'il peut introduire des polyols dans une formulation aqueuse injectable afin d'obtenir une composition iso-osmolaire.

La lidocaïne (ou chlorhydrate de lidocaïne) est un anesthésique local couramment utilisé dans les domaines esthétique et médical. Cette molécule est notamment utilisée depuis de nombreuses années dans des produits à visée esthétique comme les produits de comblement des rides afin de limiter la douleur au cours et après injection (cas du produit Zyderm® contenant du collagène et 0,3% de lidocaïne).

Dans l'art antérieur, sont décrits des gels à base d'acide hyaluronique pouvant contenir un polyol et/ou de la lidocaïne. Par exemple,
- WO 2007/077399 décrit des gels à base d'acide hyaluronique et d'un alcool biocompatible visqueux dont la stérilisation augmente la viscosité.
- WO 2004/032943 décrit des gels à base d'acide hyaluronique contenant des anesthésiques locaux dont la lidocaïne.
- WO 98/41171 décrit une composition injectable sous forme de gel à base d'acide hyaluronique, de mannitol et de lidocaïne.
- WO 2008/068297 décrit l'emploi d'un implant injectable par voie sous-cutanée ou intradermique sous forme d'hydrogel d'acide hyaluronique. Il divulgue également l'utilisation du mannitol comme agent anti-oxydant.
- European évaluation of a new hyaluronic acid filler incorporating lidocaine, G. WAHL, Journal of Cosmetic Dermatology, Vol. 7, 6, novembre 2008, pages 298-303, décrit une formulation à usage dermatologique comprenant de l'acide hyaluronique et de la lidocaïne.

On a maintenant découvert que l'ajout d'un polyol et de lidocaïne dans un gel à base d'acide hyaluronique, qu'il soit réticulé, réticulé et greffé, suivi d'une stérilisation de cette formulation à la chaleur, permet (par rapport à un gel sans polyol et sans lidocaïne):
- une amélioration très importante des propriétés rhéologiques du gel réticulé
- une amélioration de la rémanence du gel réticulé en contrant les trois grands types de dégradation d'un gel réticulé à base d'acide hyaluronique in vivo (dégradation enzymatique par les hyaluronidases, dégradation radicalaire, dégradation thermique à 37°C)
- une amélioration de la stabilité rhéologique du gel réticulé au cours du temps et donc une durée de péremption du produit pouvant être allongée II a en effet été montré que, de façon tout à fait surprenante, l'ajout d'un ou plusieurs polyol(s) et de lidocaïne dans un gel à base d'acide hyaluronique :
- ne modifie pas les propriétés rhéologiques du gel réticulé avant stérilisation à la chaleur
- modifie considérablement les propriétés rhéologiques du gel réticulé après stérilisation à la chaleur (par rapport à un gel sans polyol et sans lidocaïne)

En d'autres termes, avant stérilisation à la chaleur, les propriétés viscoélastiques d'un gel réticulé à base d'acide hyaluronique avec polyol et lidocaïne sont identiques à celles d'un gel à base d'acide hyaluronique sans polyol et sans lidocaïne.

Après stérilisation à la chaleur, les propriétés viscoélastiques d'un gel réticulé à base d'acide hyaluronique avec polyol et lidocaïne sont différentes de celles d'un gel à base d'acide hyaluronique sans polyol et sans lidocaïne. Le gel réticulé avec polyol et lidocaïne possède une plus forte élasticité (G' plus élevé) et un caractère viscoélastique plus élastique (Tanδ plus faible) par rapport à un gel sans polyol et sans lidocaïne.

La stérilisation à la chaleur modifie profondément la structure du gel réticulé et donc les propriétés viscoélastiques de celui-ci (diminution des paramètres rhéologiques G' et G" / augmentation du paramètre Tanδ). On constate que la présence d'un polyol et de la lidocaïne dans un gel réticulé à base d'acide hyaluronique modifie considérablement l'évolution des paramètres rhéologiques lors de la stérilisation à la chaleur (limitation de l'évolution des paramètres rhéologiques : diminution du G' et du G" significativement moins forte / augmentation du Tanδ significativement moins forte).

En limitant la dégradation du gel réticulé à base d'acide hyaluronique lors de la stérilisation à la chaleur, la structure du gel réticulé à base d'acide hyaluronique avec polyol et lidocaïne est différente de celle obtenue avec un gel à base d'acide hyaluronique sans polyol et sans lidocaïne. Cette structure possède notamment un caractère élastique renforcé (meilleure capacité du gel réticulé à créer du volume).

Cette meilleure résistance à la dégradation thermique confère notamment au gel réticulé une meilleure stabilité du produit à température ambiante au cours du temps. Ainsi, un produit fabriqué selon cette invention possédera une durée de péremption qui pourra être allongée par rapport à un produit ne contenant pas de polyol et de lidocaïne.

Il a également été montré qu'un gel réticulé à base d'acide hyaluronique avec polyol et lidocaïne possède une meilleure résistance aux trois facteurs de dégradation d'un gel réticulé à base d'acide hyaluronique in vivo par rapport à un gel sans polyol et sans lidocaïne :
- meilleure résistance à la dégradation enzymatique
- meilleure résistance à la dégradation radicalaire
- meilleure résistance à la dégradation thermique

En particulier, l'amélioration de la résistance à la dégradation thermique et radicalaire est tout a fait remarquable.

Sans vouloir se lier à une explication théorique de l'effet du polyol et de la lidocaïne à rencontre des dégradations d'un gel réticulé à base d'acide hyaluronique, on suppose que la lidocaïne accroit de façon considérable la capacité d'un polyol à protéger un gel réticulé à base d'acide hyaluronique.

D'autre part, le ou les polyol(s) incorporé(s) dans le gel réticulé à base d'acide hyaluronique peuvent migrer hors du gel réticulé.

Hors du gel réticulé, le ou les polyol(s) vont pouvoir diffuser dans les tissus et jouer un rôle important par exemple dans l'hydratation des tissus ou encore en intervenant dans des mécanismes cellulaires ou biochimiques.

Enfin, la présence de lidocaïne dans le gel réticulé présente un intérêt majeur pour l'amélioration du confort du patient au cours et après injection.

Par conséquent, la composition aqueuse injectable à base d'acide hyaluronique ou l'un de ses sels, d'un ou plusieurs polyol(s) et de lidocaïne selon l'invention présente les avantages suivants dans des buts esthétiques ou dans des buts thérapeutiques:
- meilleures propriétés rhéologiques de viscoélasticité du gel réticulé (notamment meilleure capacité à créer du volume) car significativement moins dégradé à la stérilisation
- plus grande rémanence du gel réticulé in vivo et donc un effet à plus long terme du traitement grâce à l'action du ou des polyol(s) et de la lidocaïne à rencontre des trois principaux types de dégradation du gel réticulé in vivo
- meilleure stabilité rhéologique du gel réticulé au cours de sa durée de péremption
- action positive des polyols sur l'organisme (par exemple, hydratation des tissus)
- amélioration du confort du patient par l'effet anesthésiant de la lidocaïne au cours et après injection.

L'invention fournit donc une composition injectable stérilisée à la chaleur comprenant un gel réticulé à base d'acide hyaluronique ou l'un de ses sels, un ou plusieurs polyol(s) et de la lidocaïne.

Cette composition est utilisée:
- dans des buts esthétiques pour le comblement des tissus biologiques ou pour la réhydratation de la peau par mésothérapie
- dans des buts thérapeutiques comme par exemple:
   a) en rhumatologie, en tant qu'agent de remplacement, de supplément temporaire du liquide synovial,
   b) en urologie/gynécologie, en tant qu'agent permettant d'augmenter le volume du sphincter ou de l'urètre,
   c) en ophtalmologie, en tant qu'adjuvant à la chirurgie de la cataracte ou pour le traitement des glaucomes,
   d) en pharmaceutique, en tant que gel réticulé à libération de substances actives,
   e) en chirurgie, pour la reconstruction osseuse, l'augmentation du volume des cordes vocales ou la fabrication de tissus chirurgicaux.

Selon les modes de réalisation de l'invention, le gel à base d'acide hyaluronique ou l'un de ses sels est réticulé.

Selon les modes de réalisation de l'invention, la concentration en acide hyaluronique ou l'un de ses sels est comprise entre 0,01 mg/ml et 100 mg/ml et la masse moléculaire de l'acide hyaluronique ou l'un de ses sels est comprise entre 1000 Da et 10*10⁶ Da.

Selon un mode de réalisation particulier de l'invention, la réticulation se fait par des molécules bi- ou poly-fonctionnelles choisies parmi les époxydes, les épihalohydrines et la divinylsulfone, sur de l'acide hyaluronique non réticulé ou déjà réticulé avec ou sans un ou plusieurs autres polysaccharides d'origine naturelle.

Les différents types de gels à base d'acide hyaluronique sont connus dans la technique, et les gels réticulés et greffés sont décrits par exemple dans WO 2005/012364.

Selon un mode particulier de l'invention, le gel réticulé peut également contenir d'autres polymères biocompatibles (comme des polysaccharides d'origine naturelle) et/ou d'autres substances actives ou non actives ayant un effet positif sur l'organisme ou sur le gel réticulé.

Selon la caractéristique de l'invention, ces gel réticulés contiennent un ou plusieurs polyol(s) choisis par exemple parmi le glycérol, le sorbitol, le propylène glycol, le xylitol, le mannitol, l'erythritol, le maltitol ou le lactitol.

Selon les modes de réalisation de l'invention, la concentration en polyol dans le gel réticulé est comprise entre 0,0001 et 500 mg/ml et plus particulièrement entre 0,0001 et 100 mg/ml.

Selon les modes de réalisation de l'invention, la concentration en lidocaïne dans le gel réticulé est comprise entre 0.0001 et 500 mg/ml et plus particulièrement entre 0,001 et 50 mg/ml.

Selon les modes de réalisation de l'invention, la stérilisation est effectuée à la chaleur sèche ou humide, préférentiellement à la chaleur humide. L'homme de l'art saura sélectionner un cycle de stérilisation à la chaleur (température et durée du cycle de stérilisation) approprié à la stérilisation de son produit. Par exemple, les cycles de stérilisation à la chaleur humide suivants peuvent être utilisés : 131°C, 1 min / 130°C, 3 min / 125°C, 7 min/121 °C, 20 min / 121°C, 10 min / 100°C, 2h.

### EXEMPLES:

Les formulations préparées dans les exemples suivants sont des gel à base de hyaluronate de sodium (NaHA) réticulé avec ou sans polyol et lidocaïne dans une solution aqueuse tamponnée à pH=7.

La préparation des gels réticulés est effectuée selon les techniques bien connues par l'homme de l'art. Le hyaluronate de sodium utilisé pour fabriquer ces gels possède une masse moléculaire égale à 2.5*10⁶ Da. Le réticulant utilisé est le butanediol diglycidyle éther (BDDE) et la définition du taux de réticulation utilisée est : masse (BDDE) / masse(NaHA sec). L'incorporation du polyol dans le gel est effectuée en ajoutant la quantité nécessaire de polyol (% exprimé en masse) dans le gel et en mélangeant à la spatule pendant 10 minutes (pour 50g de gel final).

L'incorporation de la lidocaïne dans le gel est effectuée en ajoutant la quantité nécessaire de lidocaïne (% exprimé en masse) dans le gel et en mélangeant à la spatule pendant 10 minutes (pour 50g de gel final).

Afin d'avoir des conditions de fabrication strictement identiques pour les gels avec et sans polyol et lidocaïne, le gel réticulé sans polyol et sans lidocaïne est également mélangé à la spatule pendant 10 minutes (pour 50g de gel final).

Les gels préparés sont remplis en seringue verre puis stérilisés à la chaleur (121 °C, 10 min).

Le rhéomètre utilisé pour effectuer les caractérisations rhéologiques est un AR2000 (TA instruments) avec une géométrie plate de 40 mm, un entrefer de 1000 µm et une température d'analyse de 37°C.

Exemple 1: Mise en évidence par la rhéologie de la différence de structure après stérilisation à la chaleur entre des gels à base d'acide hyaluronique avec et sans polyol/lidocaïne.

Soit A, un gel à base de NaHA réticulé (Masse Molaire NaHA = 2,5x10⁶ Da, concentration en NaHA = 22,5 mg/ml, Taux de réticulation = 9%). Le gel est ensuite purifié par dialyse pendant 24 heures (cellulose régénérée, limite de séparation : Masse Molaire = 6OkDa). 150g de gel purifié est mélangé 10 minutes à la spatule.

Le gel ainsi obtenu est divisé en trois fractions de masse égale (50g).

Soit B, la fraction n°1. Dans cette fraction, on ajoute 1 % de glycérol, 1 ,5% de sorbitol et 0.3% de lidocaïne. Le gel est mélangé à la spatule pendant 10 minutes.

Soit C, la fraction n° 2. Dans cette fraction, on ajoute 1 % de glycérol et 1 ,5% de sorbitol. Le gel est mélangé à la spatule pendant 10 minutes.

Soit D, la fraction n°3. On ajoute une solution de NaCl de concentration adaptée afin d'avoir une concentration en acide hyaluronique et une osmolarité équivalentes à celles des gels B et C. Le gel est mélangé à la spatule pendant 10 minutes.

Soient B, C et D, les gels issus des fractions B, C et D respectivement.

Les gels ainsi obtenus ont un pH proche de 7,00, une osmolarité proche de 300 mOsm/kg et une concentration en acide hyaluronique équivalente.

Une mesure de rhéologie (balayage en fréquence - 0.01 à 100 Hz) est effectuée pour chacun des gels B, C et D avant stérilisation.

Une comparaison des valeurs de G' (= module élastique), G" (= module visqueux) et Tanδ=G"/G' est effectuée à 1 Hz.

| Formulation | G' (1Hz) (Pa) | G" (1Hz) (Pa) | Tanδ( 1 Hz) |
|---|---|---|---|
| Gel B (avant stérlisation) | 316 | 94 | 0,297 |
| Gel C (avant stérlisation) | 314 | 93 | 0,296 |
| Gel D (avant stérlisation) | 318 | 94 | 0,296 |

On ne constate pas de différences rhéologiques, donc de différence de structure entre les trois gels B, C et D avant stérilisation.

Les gels B, C et D sont remplis en seringue verre 1 ml puis stérilisés à la chaleur humide à 121°C pendant 10 minutes.

Une mesure de rhéologie (balayage en fréquence - 0.01 à 100 Hz) est effectuée pour chacun des gels B, C et D après stérilisation.

Une comparaison des valeurs de G' (= module élastique), G" (= module visqueux) et Tanδ=G"/G' est effectuée à 1 Hz.

| Formulation | G'(1Hz) (Pa) | G"(1Hz) (Pa) | Tanδ(1Hz) |
|---|---|---|---|
| Gel B (après stérilisation) Selon l'invention | 128 | 62 | 0,484 |
| Gel C (après stérilisation) | 91 | 47 | 0,516 |
| Gel D (après stérilisation) | 75 | 50 | 0,667 |

On constate des différences rhéologiques importantes, donc des différences importantes de structure entre les trois gels B, C et D après stérilisation à la chaleur.

Après stérilisation à la chaleur, le gel selon l'invention (= gel B) présente une élasticité (G' plus élevé) et un caractère élastique (Tanδ plus faible) significativement plus important que le gel sans polyol et sans lidocaïne (= gel D).

La présence des polyols et de la lidocaïne a permis de fortement limiter la dégradation thermique du gel à base d'acide hyaluronique au cours de la stérilisation à la chaleur.

| Formulation | Variation G'(1Hz) avant/après stérilisation |
|---|---|
| Gel B | - 59% |
| Selon l'invention | |
| Gel C | - 71% |
| Gel D | - 76% |

Exemple 2: Mise en évidence de la meilleure résistance à la dégradation enzymatique d'un gel à base d'acide hyaluronique avec polyol et lidocaïne.

La résistance à la dégradation enzymatique du gel B selon l'invention (voir exemple 1) est comparée à celle du gel D (voir exemple 1). Le test de dégradation est effectué à l'aide d'un rhéomètre AR2000 (TA instruments) avec une géométrie plate de 40 mm et un entrefer de 1000 µm.

Le test de dégradation est effectué en ajoutant une solution de hyaluronidases dans le gel à tester, en homogénéisant à la spatule pendant 1 minute, en se plaçant à la température de 37°C et en imposant une déformation de 0,3%. La valeur du paramètre G' à 1 Hz est mesurée à t=5 min et t=40 min.

| Formulation | G'(1Hz) (Pa) At = 5 min | G'(1Hz) (Pa) At = 40 min | ΔG'(1 Hz) |
|---|---|---|---|
| Gel B (stérile) | 115 | 52 | - 55% |
| Selon l'invention | | | |
| Gel D (stérile) | 60 | 24 | - 60% |

On constate que le gel selon l'invention a une meilleure résistance à la dégradation enzymatique.

Exemple 3: Mise en évidence de la meilleure résistance à la dégradation radicalaire d'un gel à base d'acide hyaluronique avec polyol et lidocaïne.

La résistance à la dégradation radicalaire du gel B selon l'invention (voir exemple 1), du gel C (voir exemple 1) et du gel D (voir exemple 1) est comparée. Le test de dégradation est effectué à l'aide d'un rhéomètre AR2000 (TA instruments) avec une géométrie plate de 40 mm et un entrefer de 1000 µm.

Le test de dégradation est effectué en ajoutant un oxydant dans le gel à tester, en homogénéisant à la spatule pendant 1 minute, en se plaçant à la température de 37°C et en imposant une déformation de 0,3%. La valeur du paramètre G' à 1 Hz est mesurée à t=5 min et t=40 min.

| Formulation | G'(1Hz) (Pa) At = 5 min | G'(1Hz) (Pa) At = 40 min | ΔG'(1 Hz) |
|---|---|---|---|
| Gel B (stérile) | 122 | 98 | - 20% |
| Selon l'invention | | | |
| Gel C (stérile) | 80 | 38 | - 53% |
| Gel D (stérile) | 58 | 20 | - 66% |

On constate que le gel selon l'invention possède une bien meilleure résistance à la dégradation radicalaire.

Exemple 4: Mise en évidence de la meilleure résistance à la dégradation thermique d'un gel à base d'acide hyaluronique avec polyol et lidocaïne La résistance à la dégradation thermique du gel B selon l'invention (voir exemple 1) et du gel D (voir exemple 1) est comparée.

Le test de dégradation thermique est effectué en introduisant le gel à tester dans une étuve à 80°C pendant 8 jours et en mesurant la valeur du paramètre G'(1 Hz) à t=0 et à t=8 jours.

| Formulation | G'(1 Hz) (Pa) At = 0 | G'(1Hz) (Pa) At = 8 jours | ΔG'(1 Hz) |
|---|---|---|---|
| Gel B (stérile) | 128 | 66 | - 48% |
| Selon l'invention | | | |
| Gel D (stérile) | 75 | 29 | - 61% |

On constate que le gel selon l'invention a une meilleure résistance à la dégradation thermique.

Exemple 5: Mise en évidence de la meilleure stabilité rhéologique à température ambiante au cours du temps d'un gel à base d'acide hyaluronique avec polyol et lidocaïne

Le gel B selon l'invention (voir exemple 1) et le gel D (voir exemple D) ont été stockés 8 mois à température ambiante (25°C).

La valeur du paramètre G'(1 Hz) a été mesurée à t=0, t=4 mois et t=8 mois.

| Formulation | Gel B (stérile) | Gel D (stérile) |
|---|---|---|
| | Selon l'invention | |
| G'(1 Hz) (Pa) | 128 | 75 |
| At = 0 | | |
| G'(1Hz) (Pa) | 126 | 68 |
| At = 4 mois | | |
| G'(1Hz) (Pa) | 120 | 66 |
| At = 8 mois | | |
| ΔG'(1 Hz) | - 6% | -12% |
| (0 - 8 mois) | | |

3On constate que le gel selon l'invention a une meilleure stabilité rhéologique à température ambiante au cours du temps.

## Revendications

1. Composition aqueuse injectable sous forme de gel, utilisée dans des buts esthétiques ou dans des buts thérapeutiques, à base d'acide hyaluronique ou l'un de ses sels, d'un ou plusieurs polyol(s) et de lidocaïne, ayant subi une stérilisation à la chaleur qui a pour effets une amélioration des propriétés rhéologiques de viscoélasticité et une amélioration de la rémanence in vivo, par rapport à un gel sans polyol et sans lidocaïne, **caractérisée en ce que** le gel à base d'acide hyaluronique ou l'un de ses sels est réticulé.

2. Composition aqueuse injectable selon la revendication 1, **caractérisée en ce que** la stérilisation est effectuée à la chaleur humide.

3. Composition aqueuse injectable selon la revendication 1, **caractérisée en ce que** la concentration en acide hyaluronique ou l'un de ses sels est comprise entre 0,01 mg/ml et 100 mg/ml, et la masse moléculaire de l'acide hyaluronique ou l'un de ses sels est comprise entre 1000 Da et 10 x 10⁶ Da.

4. Composition aqueuse injectable selon l'une quelconque des revendications 1 à 3, **caractérisée en ce que** la concentration en polyol est comprise entre 0,0001 et 100 mg/ml.

5. Composition aqueuse injectable selon l'une quelconque des revendications 1 à 4, **caractérisée en ce que** la concentration en lidocaïne est comprise entre 0,0001 et 50 mg/ml.

6. Composition aqueuse injectable selon l'une quelconque des revendications 1 à 5, **caractérisée en ce que** le gel à base d'acide hyaluronique ou l'un de ses sels est réticulé avec ou sans un ou plusieurs autres polysaccharides d'origine naturelle.

7. Composition aqueuse injectable selon la revendications 1 à 6, **caractérisée en ce que** le gel à base d'acide hyaluronique ou l'un de ses sels est réticulé par des molécules bi- ou poly-fonctionnelles choisies parmi les époxydes, les épihalohydrines et la divinylsulfone.

8. Composition aqueuse injectable selon l'une quelconque des revendications 1 à 7, **caractérisée en ce que** le polyol est choisi par exemple parmi le glycérol, le sorbitol, le mannitol, le propylène glycol, l'érythritol, le xylitol, le maltitol et le lactitol.

9. Composition aqueuse injectable selon l'une quelconque des revendications 1 à 8, utilisée pour le comblement ou le remplacement de tissus biologiques.

10. Composition aqueuse injectable selon l'une quelconque des revendications 1 à 8, utilisée pour le comblement des rides, le remodelage du visage ou l'augmentation du volume des lèvres.

11. Composition aqueuse injectable selon l'une quelconque des revendications 1 à 8, utilisée dans le traitement de réhydratation de la peau par mésothérapie.

12. Composition aqueuse injectable selon l'une quelconque des revendications 1 à 8, utilisée pour séparer, remplacer ou combler un tissu biologique ou augmenter le volume dudit tissu.

13. Composition aqueuse injectable selon l'une quelconque des revendications 1 à 8, utilisée en:
- en rhumatologie, en tant qu'agent de remplacement, de supplément temporaire du liquide synovial;
- en urologie/gynécologie, en tant qu'agent permettant d'augmenter le volume du sphincter ou de l'urètre;
- en ophtalmologie, en tant qu'adjuvant à la chirurgie de la cataracte ou pour le traitement des glaucomes;
- en pharmaceutique, en tant que gel à libération de substances actives;
- en chirurgie, pour la reconstruction osseuse, l'augmentation du volume des cordes vocales ou la fabrication de tissus chirurgicaux.

## Patentansprüche

1. Injizierbare wässrige Zusammensetzung in Gelform, die zu ästhetischen Zwecken oder therapeutischen Zwecken verwendet wird, auf der Basis von Hyaluronsäure oder eines ihrer Salze, eines oder mehrerer Polyol/s/e und von Lidocain, die mit Wärme sterilisiert wurde und die im Vergleich zu einem Gel ohne Polyol und ohne Lidocain eine Verbesserung der rheologischen Viskoelastizitätseigenschaften und eine Verbesserung der in vivo-Remanenz zur Folge hat, **dadurch gekennzeichnet, dass** das Gel auf der Basis von Hyaluronsäure oder eines ihrer Salze vernetzt ist.

2. Injizierbare wässrige Zusammensetzung nach Anspruch 1, **dadurch gekennzeichnet, dass** die Sterilisierung mit feuchter Wärme durchgeführt wird.

3. Injizierbare wässrige Zusammensetzung nach Anspruch 1, **dadurch gekennzeichnet, dass** die Konzentration der Hyaluronsäure oder eines ihrer Salze zwischen 0,01 mg/ml und 100 mg/ml inklusive ist und die molekulare Masse der Hyaluronsäure oder eines ihrer Salze zwischen 1000 Da und 10 x 10⁶ Da inklusive ist.

4. Injizierbare wässrige Zusammensetzung nach einem der Ansprüche 1 bis 3, **dadurch gekennzeichnet, dass** die Polyolkonzentration zwischen 0,0001 und 100 mg/ml ist.

5. Injizierbare wässrige Zusammensetzung nach einem der Ansprüche 1 bis 4, **dadurch gekennzeichnet, dass** die Lidocainkonzentration zwischen 0,0001 und 50 mg/ml ist.

6. Injizierbare wässrige Zusammensetzung nach einem der Ansprüche 1 bis 5, **dadurch gekennzeichnet, dass** das Gel auf der Basis von Hyaluronsäure oder eines ihrer Salze mit oder ohne einem oder mehreren anderen natürlichen Polysacchariden vernetzt ist.

7. Injizierbare wässrige Zusammensetzung nach den Ansprüchen 1 bis 6, **dadurch gekennzeichnet, dass** das Gel auf der Basis von Hyaluronsäure oder eines ihrer Salze durch bi- oder multifunktionelle Moleküle vernetzt ist, die aus den Epoxyden, den Epihalohydrinen und dem Divinylsulfon ausgewählt sind.

8. Injizierbare wässrige Zusammensetzung nach einem der Ansprüche 1 bis 7, **dadurch gekennzeichnet, dass** das Polyol beispielsweise aus dem Glycerol, dem Sorbitol, dem Mannitol, dem Propylenglycol, dem Erythritol, dem Xylitol, dem Maltitol und dem Lactitol ausgewählt ist.

9. Injizierbare wässrige Zusammensetzung nah einem der Ansprüche 1 bis 8, die zum Aufpolstern oder zum Ersatz biologischer Gewebe verwendet wird.

10. Injizierbare wässrige Zusammensetzung nach einem der Ansprüche 1 bis 8, die zum Aufpolstern von Falten, zur Modellierung des Gesichts oder zur Vergrößerung des Volumens der Lippen verwendet wird.

11. Injizierbare wässrige Zusammensetzung nach einem der Ansprüche 1 bis 8, die für die Rehydrationsbehandlung der Haut durch Mesotherapie verwendet wird.

12. Injizierbare wässrige Zusammensetzung nach einem der Ansprüche 1 bis 8, die verwendet wird, um ein biologisches Gewebe zu separieren, zu ersetzen oder aufzupolstern oder um das Volumen des Gewebes zu vergrößern.

13. Injizierbare wässrige Zusammensetzung nach einem der Ansprüche 1 bis 8, die verwendet wird:
- in der Rheumatologie als Ersatz-, temporäres Ergänzungsmittel der Gelenkflüssigkeit,
- in der Urologie/Gynäkologie als Mittel, das es erlaubt, das Volumen des Schließmuskels oder der Harnröhre zu vergrößern,
- in der Augenheilkunde als Adjuvans in der Kataraktchirurgie oder zur Behandlung von Glaukomen,
- in der Pharmazie als Gel für die Freisetzung aktiver Substanzen,
- in der Chirurgie für die Wiederherstellung von Knochen, die Vergrößerung des Volumens der Stimmbänder oder die Herstellung von chirurgischen Geweben.

## Claims

1. An injectable aqueous composition as a gel, used for aesthetical purposes or therapeutical purposes, based on hyaluronic acid or one of its salts, on one or more polyol(s) and on lidocain, having undergone heat sterilization which has the effects of improving visco-elastic rheological properties and improving in vivo persistence, as compared with a gel without any polyol and without any lidocaine, **characterized in that** the gel based on hyaluronic acid or one of its salts is cross-linked.

2. The injectable aqueous composition according to claim 1, **characterized in that** sterilization is carried out with humid heat.

3. The injectable aqueous composition according to claim 1, **characterized in that** the concentration of hyaluronic acid or one of its salts is comprised between 0.01 mg/ml and 100 mg/ml, and the molecular weight of hyaluronic acid or one of its salts is comprised between 1,000 Da and 10 x 10⁶ Da.

4. The injectable aqueous composition according to any of claims 1 to 3, **characterized in that** the polyol concentration is comprised between 0.0001 and 100 mg/ml.

5. The injectable aqueous composition according to any of claims 1 to 4, **characterized in that** the lidocain concentration is comprised between 0.0001 and 50 mg/ml.

6. The injectable aqueous composition according to any of claims 1 to 5, **characterized in that** the gel based on hyaluronic acid or one of its salts is cross-linked with or without one or more other polysaccharide(s) of natural origin.

7. The injectable aqueous composition according to claims 1 to 6, **characterized in that** the gel based on hyaluronic acid or one of its salts is cross-linked with bi- or poly-functional molecules selected from epoxides, epihalohydrins and divinylsulfone.

8. The injectable aqueous composition according to any of claims 1 to 7, **characterized in that** the polyol is for example selected from glycerol, sorbitol, mannitol, propylene glycol, erythritol, xylitol, maltitol and lactitol.

9. The injectable aqueous composition according to any of claims 1 to 8, used for filling or replacing biological tissues.

10. The injectable aqueous composition according to any of claims 1 to 8, used for filling wrinkles, remodeling the face or increasing the volume of the lips.

11. The injectable aqueous composition according to any of claims 1 to 8, used in skin rehydration treatment by mesotherapy.

12. The injectable aqueous composition according to any of claims 1 to 8, used for separating, replacing, or filling a biological tissue or increasing the volume of said tissue.

13. The injectable aqueous composition according to any of claims 1 to 8, used:
- in rheumatology, as a temporary replacement, supplemental agent of synovial fluid;
- in urology/gynecology, as an agent allowing increase in the volume of the sphincter or urethra;
- in ophthalmology, as an adjuvant to cataract surgery or for treating glaucomas;
- in pharmaceutics, as a gel for releasing active substances;
- in surgery, for bone reconstruction, increasing the volume of the vocal chords or for manufacturing surgical tissues.
